# FASCICULE DE BREVET EUROPEEN

(11) **EP 0 488 904 B1**
(45) Date de publication et mention de la délivrance du brevet: **26.04.1995**
(21) Numéro de dépôt: 91403251.1
(22) Date de dépôt: 29.11.1991
(51) Int. Cl.: A61N 1/368

(54) **Procédé de commande d'un stimulateur cardiaque de type DDD, à commutation automatique de mode de fonctionnement**
Verfahren zur Steuerung eines mit automatischer Umschaltung der Betriebsmode versehenen Herzschrittmachers von Typ DDD
Method for controlling a pacemaker of type DDD with automatic switching of the operation mode

(30) Priorité: 30.11.1990 FR 9015013
(43) Date de publication de la demande: 03.06.1992
(73) Titulaire: ELA-MEDICAL (Société Anonyme), F-92541 Montrouge (FR)
(72) Inventeur: Girodo, Sylvie, F-92120 Montrouge (FR); Malherbe, Odile, F-94230 Cachan (FR); Limousin, Marcel, F-92120 Montrouge (FR)
(74) Mandataire: Laget, Jean-Loup

(56) Documents cités:
- EP-A- 0 238 966
- EP-A- 0 360 668
- US-A- 4 944 298
- THE AMERICAN JOURNAL OF CARDIOLOGY vol. 52, no. 1, Juillet 1983, pages 88 - 91; RUBIN ET AL: 'Current physiologic pacemakers : a serious problem with a new device'

## Description

L'invention concerne un procédé de commande d'un stimulateur cardiaque de type DDD, à commutation automatique de mode de fonctionnement.

Les dysfonctions sinusales isolées, ou entrant dans le cadre de la maladie de l'oreillette, posent un problème quant au choix du mode de stimulation : la stimulation auriculaire simple chambre, correspondant au mode de fonctionnement AAI du stimulateur, paraît la plus appropriée. Cependant, lorsque la déficience du sinus s'étend au faisceau de His, on constate l'apparition de blocs auriculo-ventriculaires paroxystiques et corrélativement le défaut de dépolarisation du ventricule. Il faut donc procéder à une stimulation du ventricule. Par mesure de sécurité, il est usuel dans de telles circonstances, d'implanter un stimulateur double chambre, fonctionnant en mode DDD. Dans ce cas, le stimulateur stimule le ventricule systématiquement alors qu'en dehors des périodes de crise, la stimulation du ventricule est inutile.

Un but de la présente invention est de proposer un stimulateur cardiaque capable d'assurer une stimulation du ventricule en période de crise seulement.

Un autre but de l'invention est de proposer un stimulateur cardiaque de type DDD, fonctionnant en mode DDD pendant les périodes de crise seulement, et en mode AAI en dehors de ces périodes de crise.

Un autre but encore de l'invention est de proposer un stimulateur à commutation de mode de fonctionnement, qui fonctionne avec des paramètres automatiquement optimisés sans qu'il soit nécessaire de les programmer.

L'invention a pour objet un procédé de commande d'un stimulateur cardiaque de type DDD, à commutation automatique de mode de fonctionnement, caractérisé en ce que :
- on contrôle le coeur en mode AAI pendant les périodes de conduction auriculo-ventriculaire ,
- après chaque événement auriculaire hors des périodes réfractaires, on déclenche un délai de surveillance ventriculaire,
- en cas de défaut de conduction auriculo-ventriculaire pendant ledit délai, on bascule automatiquement en mode DDD et on déclenche une stimulation ventriculaire,
- après rétablissement de la conduction auriculo-ventriculaire, on rebascule automatiquement en mode AAI.

Selon d'autres caractéristiques de l'invention :
Le délai de surveillance ventriculaire est égal à la moyenne des intervalles entre un événement auriculaire et la détection ventriculaire suivante au cours des cycles précédents augmentée d'une durée prédéterminée, de façon à favoriser la conduction auriculo-ventriculaire spontanée ;
Le délai de surveillance ventriculaire est égal à la moyenne des intervalles entre un événement auriculaire et la détection ventriculaire suivante au cours des huit cycles précédents et la durée prédéterminée est comprise entre 16 et 78ms et de préférence égale à 47ms ;
Le délai de surveillance ventriculaire est limité à 350ms ;
Lors du basculement en mode DDD, le délai auriculo-ventriculaire est calculé par interpolation linéaire entre une valeur maximale et une valeur minimale, en fonction du rythme sinusal ;
Les valeurs maximale et minimale du délai auriculo-ventriculaire sont mémorisées pendant les périodes de conduction auriculo-ventriculaire spontanée ;
Lorsque la valeur maximale DAVmax est inférieure à la valeur de l'intervalle ER moyen sur un certain nombre de cycles de préférence égal à 8, alors cette valeur DAVmax est augmentée par pas de 16ms ;
Lorsque, pendant une durée déterminée, typiquement 24h, la valeur DAVmax n'a pas varié, alors elle est diminuée de 16ms ;
Lorsque la valeur minimale DAVmin est supérieure à la valeur de l'intervalle ER moyen sur un certain nombre de cycles de préférence égal à 8, alors cette valeur DAVmin est diminuée par pas de 16 ms;
Lorsque pendant une durée déterminée, typiquement 24 h, la valeur DAVmin n'a pas varié, alors elle est augmentée de 16 ms.

Chaque fois que l'on passe d'une détection auriculaire à une stimulation auriculaire, on calcule une extension du délai auriculo-ventriculaire, égale à la différence entre d'une part l'intervalle entre la stimulation auriculaire et la détection ventriculaire suivante, et d'autre part l'intervalle entre la détection auriculaire et la détection ventriculaire suivante, ce dernier intervalle ayant été mesuré au cours de l'antépénultième cycle ;
Chaque fois que l'on passe d'une stimulation auriculaire à une détection auriculaire, on calcule une extension du délai auriculo-ventriculaire, égale à la différence entre d'une part l'intervalle entre la stimulation auriculaire et la détection ventriculaire suivante, et d'autre part la détection auriculaire et la détection ventriculaire suivante, le premier intervalle ayant été mesuré au cours de l'antépénultième cycle ;
Les conditions du rétablissement de la conduction auriculo-ventriculaire sont réunies lorsque l'une des conditions suivantes est réalisée :
- passage d'une activité auriculaire stimulée à une détection auriculaire,
- récupération d'une activité ventriculaire spontanée,
- après cent cycles avec stimulation ventriculaire ;
Le rebasculement en mode AAI est automatiquement assuré si, pendant seize cycles consécutifs, le stimulateur détecte des ondes R synchrones des événements auriculaires ;
Lorsque la fréquence de stimulation, ou la fréquence spontanée, devient inférieure ou égale à une fréquence de référence prédéterminée par rapport à la fréquence de base, ladite fréquence de référence prédéterminée correspondant à un intervalle d'échappement égal à l'intervalle d'échappement de base diminué d'une durée égale de préférence à 31 ms, alors pour favoriser le rétablissement de la conduction auriculo-ventriculaire le délai AV est prolongé d'une durée comprise entre 16 et 78 ms et de préférence égale à 47 ms.

Lors du passage de détection auriculaire à stimulation auriculaire, l'intervalle d'échappement auriculaire est raccourci, pendant au plus huit cycles, d'une valeur programmée, pour accélérer la fréquence de stimulation auriculaire de façon à compenser la chute du débit sanguin.

D'autres caractéristiques ressortent de la description qui suit faite avec référence aux dessins annexés sur lesquels on peut voir :
Fig 1 un schéma représentatif des signaux cardiaques en cas de détection auriculaire, montrant le passage au mode de fonctionnement DDD à la première stimulation du ventricule.
Fig. 2 un schéma représentatif des signaux cardiaques en cas de stimulation de l'oreillette montrant le passage au mode de fonctionnement DDD à la première stimulation du ventricule.
Fig. 3 un schéma explicatif de l'extension du DAV.
Fig. 4 un schéma représentatif de différentes périodes calculées par le stimulateur en mode de fonctionnement AAI.
Fig. 5 un schéma représentatif de différentes périodes calculées par le stimulateur lors du passage du mode de fonctionnement AAI au mode de fonctionnement DDD.

Selon la présente invention, un stimulateur de type DDD est implanté chez les patients ayant une indication de stimulation auriculaire et chez lesquels on peut craindre, en outre, un trouble de conduction auriculo-ventriculaire.

Ce stimulateur fonctionne en mode DDD automatique, c'est-à-dire qu'en dehors des périodes de crise, il fonctionne en mode AAI, et il passe automatiquement en mode DDD lors de l'apparition d'un trouble de conduction auriculo-ventriculaire, et il repasse automatiquement en mode AAI lorsque l'une de certaines conditions est remplie.

En pratique, lorsque le mode DDD automatique est utilisé, le stimulateur propose un fonctionnement en mode AAI qui permet à la conduction spontanée de s'exprimer. Mais dès qu'un ralentissement de la conduction apparait, le stimulateur bascule en mode DDD et fonctionne avec des paramètres automatiquement optimisés sans qu'il soit nécessaire de les programmer.

Le stimulateur fonctionne en mode AAI tant que la conduction auriculo-ventriculaire est normale, c'est-à-dire tant que chaque événement auriculaire (hors des périodes réfractaires) est suivi d'une détection ventriculaire synchrone. Par événement auriculaire, il faut comprendre soit une détection auriculaire soit une stimulation auriculaire.

Pour détailler le comportement du stimulateur dans cette situation, il faut distinguer deux cas, selon que l'activité auriculaire est spontanée ou stimulée.

On appelle événement auriculaire E, soit une détection auriculaire P, soit une stimulation auriculaire A, ouvrant respectivement un intervalle PR ou AR, noté de façon générale ER.

### Détection auriculaire.

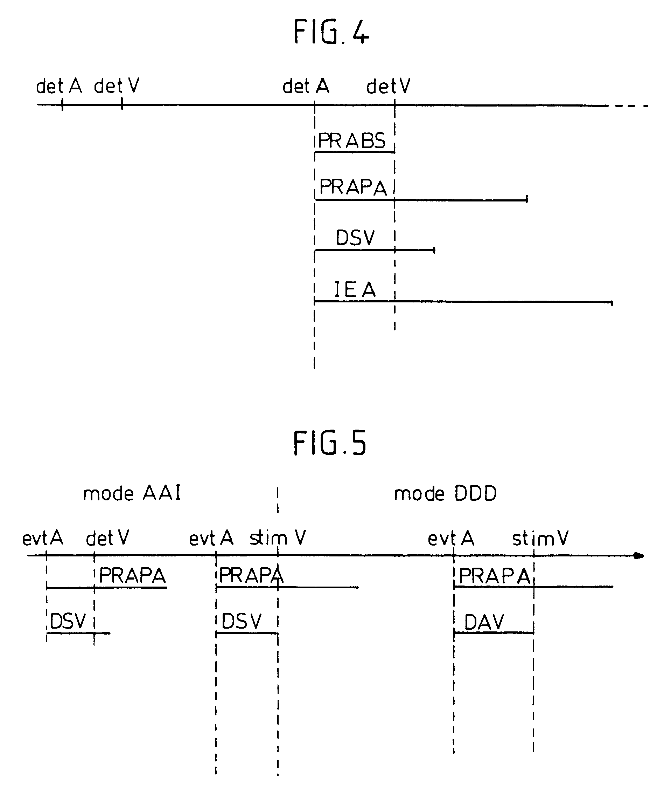

Tant que la conduction auriculo-ventriculaire est normale, et sur toute détection auriculaire, le stimulateur déclenche différentes périodes (Fig4) :
- une période réfractaire absolue auriculaire (PRABS), qui inhibe la détection auriculaire jusqu'à l'événement ventriculaire suivant ;
- une période réfractaire auriculaire post-auriculaire (PRAPA), comprise entre 62,5 et 87,5% et de préférence égale à 75% de l'intervalle PP du cycle précédent ; toute onde P survenant pendant cette période est considérée comme une extrasystole auriculaire qui peut ne pas être conduite dans le ventricule sans que l'on puisse pour autant parler d'un trouble de conduction, cette onde P fait démarrer un nouvel intervalle d'échappement auriculaire, mais n'a pas d'autre influence sur le fonctionnement du stimulateur ;
- un délai de surveillance ventriculaire (DSV), équivalent au délai auriculo-ventriculaire (DAV) du mode DDD classique, mais égal à la moyenne des 8 intervalles ER spontanés précédents augmentée d'une valeur comprise entre 16 et 78ms et de préférence de 47ms : cette période permet de surveiller la conduction auriculo-ventriculaire ;
- un intervalle d'échappement auriculaire (IEA), dont la valeur est calculée en fonction d'une moyenne des intervalles PP sur 8 cycles par l'algorithme de lissage.

### Stimulation auriculaire.

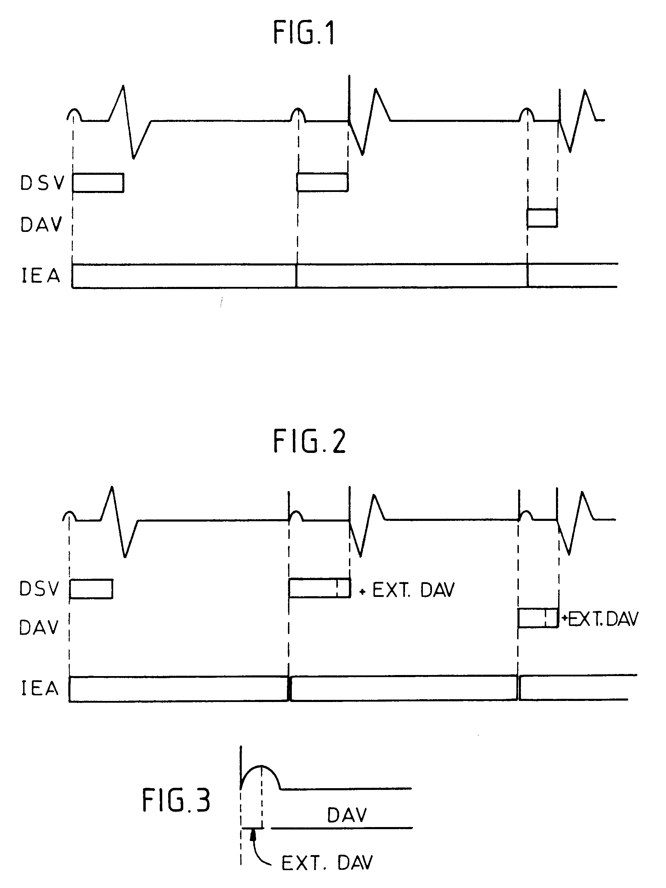

Tant que la conduction auriculo-ventriculaire est normale, et sur toute stimulation auriculaire, le stimulateur déclenche les mêmes périodes que celles décrites dans le paragraphe précédent, mais de durées différentes :
- la période réfractaire auriculaire post-auriculaire (PRAPA) est égale à 75% de la moyenne des 8 intervalles PP précédents,
- le délai de surveillance ventriculaire (DSV) est égal à l'intervalle ER moyen entre un événement auriculaire et la détection ventriculaire suivante sur les 8 cycles précédents, augmenté d'une durée comprise entre 16 et 78ms et de préférence égale à 47ms et de l'extension du DAV (Fig 2) ;
- l'intervalle d'échappement auriculaire est égal à la valeur calculée par l'algorithme de lissage.

Tant que les conductions auriculaire et auriculo-ventriculaire sont normales, le stimulateur calcule automatiquement la valeur de certains paramètres. Ainsi, quand ces paramètres sont utilisés, le fonctionnement de l'appareil est optimisé par l'utilisation de valeurs adaptées à la physiologie du patient.

Ces paramètres sont le délai auriculo-ventriculaire de base (DAV de base), le délai auriculo-ventriculaire minimal (DAV minimal), l'extension du DAV et l'intervalle d'échappement auriculaire (IEA)

L'algorithme d'ajustement automatique du DAV en fonction de la fréquence sinusale détectée utilise deux paramètres pour fonctionner : les DAV de base et minimal. Quand le mode DDD automatique est programmé, et que le rythme est sinusal, ces deux paramètres sont automatiquement calculés par le stimulateur, bien qu'ils n'interviennent à aucun moment tant que le stimulateur fonctionne en mode AAI. Les valeurs calculées seront prises en compte quand le stimulateur aura basculé en mode DDD, à la suite de l'apparition d'un trouble de conduction auriculo-ventriculaire.

### DAV de base.

Le DAV de base ou DAVmax reste toujours compris entre 256 et 156ms.

Au moment où le mode DDD automatique est programmé, la valeur DAVmax, du paramètre DAV de base est initialisée à son minimum, par exemple 156ms.

La valeur DAVmax est comparée à la valeur de l'intervalle ER moyen sur 8 cycles.

Lorsque la valeur DAVmax est inférieure à la valeur de l'intervalle ER moyen sur 8 cycles, alors la valeur DAVmax est augmentée par pas de 16ms (deux valeurs calculées consécutivement ne peuvent différer de plus de 16ms).

Si pendant une durée déterminée, typiquement 24h, la valeur DAVmax ne varie pas, alors elle est diminuée de 16 ms.

### DAV minimal.

La valeur DAVmin de ce paramètre ne peut pas être inférieure à 78ms.

Au moment où le mode DDD automatique est programmé, la valeur DAVmin du paramètre DAV minimal est initialisée à son maximum, par exemple 141ms.

La valeur DAVmin est comparée à la valeur de l'intervalle ER moyen sur 8 cycles.

Lorsque la valeur DAVmin est supérieure à ER moyen sur 8 cycles, alors la valeur DAVmin est diminuée par pas de 16ms.

Si pendant une durée déterminée, typiquement 24 h, la valeur DAVmin ne varie pas, alors elle est augmentée de 16 ms.

Le DAV qui sera utilisé en mode DDD est calculé par interpolation linéaire entre la valeur maximale et la valeur minimale, en fonction du rythme sinusal.

### Extension du DAV.

L'extension du DAV est une valeur qui s'ajoute au DAV après chaque stimulation auriculaire, et permet de compenser le retard à la détection dans l'oreillette par rapport à la stimulation (Fig 3). En mode AAI, sa valeur nominale est typiquement 31ms.

Lors de la programmation du mode DDD automatique, la valeur de l'extension du DAV, appelée aussi hystérésis du DAV, est initialisée à 78ms. Elle est recalculée chaque fois que l'on passe d'une détection auriculaire à une stimulation auriculaire : elle est égale à la différence entre d'une part l'intervalle entre la stimulation auriculaire et la détection ventriculaire suivante, et d'autre part l'intervale PR mesuré au cours de l'antépénultième cycle. A chaque recalcul, son pas de variation est limité à 16ms.

Si l'on passe d'une stimulation auriculaire à une détection auriculaire, on peut faire le même calcul en inversant les intervalles. Le saut d'un intervalle au moment du passage de la détection à la stimulation auriculaire, ou inversement, permet d'éviter une erreur de calcul due à un éventuel chevauchement entre stimulation et dépolarisation spontanée de l'oreillette.

Chaque fois que l'intervalle ER diminue de plus de 31ms sur un cycle par rapport à la moyenne sur 8 cycles, le recalcul de l'extension du DAV, ou hystérésis du DAV, est inhibé.

La valeur de l'extension du DAV reste toujours comprise entre 31 et 78ms.

### Intervalle d'échappement auriculaire (IEA).

La programmation du mode DDD automatique entraîne obligatoirement la programmation systématique de l'algorithme de lissage, même si celui-ci n'était pas utilisé auparavant. L'intervalle d'échappement auriculaire est donc calculé par cet algorithme : il est modifié tous les 8 cycles, de façon à être en permanence un peu plus long que la moyenne des intervalles PP des 8 cycles précédents.

En cas de ralentissement rapide de l'activité sinusale, la première stimulation auriculaire est délivrée à la fin de l'intervalle d'échappement auriculaire (Fig 2). Puis cet intervalle d'échappement est allongé de la valeur programmée pour la pente de décélération, une fois tous les 8 cycles, de façon à ramener lentement la fréquence de stimulation auriculaire à la fréquence de base, si la détection d'une activité sinusale spontanée n'est pas retrouvée entretemps.

Par ailleurs, dans les cycles qui suivent la première stimulation auriculaire, l'intervalle d'échappement auriculaire peut être raccourci d'une valeur programmable, appelée pente d'accélération : ceci a pour effet d'accélérer la fréquence de stimulation auriculaire juste après l'arrêt sinusal (pendant 8 cycles au maximum), de façon à tenter de compenser la baisse d'inotropisme induite par l'hyper-réflectivité vagale (syndrome du sinus carotidien et syndrome vaso-vagal). Cette pente d'accélération est un paramètre programmable, qui peut aussi prendre une valeur nulle : dans ce cas, il n'y a pas d'accélération de la fréquence de stimulation, et, après huit cycles, l'intervalle d'échappement auriculaire est allongé de la valeur de la pente de décélération, comme décrit dans le paragraphe précédent.

Si l'algorithme de lissage était déjà utilisé au moment où le mode DDD automatique est programmé, il continue de fonctionner comme auparavant. Par contre, si ce n'était pas le cas, la programmation du mode DDD automatique force la programmation du lissage, avec une pente de décélération fixée à 31ms par défaut. La pente d'accélération est un paramètre indépendant qui doit être volontairement programmé pour entrer en fonctionnement (sa valeur par défaut est nulle).

En se reportant à la Fig. 1, on voit à gauche les signaux physiologiques d'un coeur contrôlé par le stimulateur fonctionnant en mode AAI : la dépolarisation ventriculaire intervient avant la fin du délai de surveillance du ventricule (DSV). Au milieu de la Fig.1, on voit que le ventricule est stimulé à la fin du DSV. Cette stimulation correspond au basculement du stimulateur en mode DDD, qui se poursuit à la partie droite de la Fig. 1.

Sur la Fig. 2, la partie gauche correspond au fonctionnement physiologique du coeur contrôlé par un stimulateur fonctionnant en mode AAI. Au milieu de la Fig. 2, on voit que l'oreillette est stimulée à la fin de l'intervalle d'échappement auriculaire, par le stimulateur qui fonctionne toujours en mode AAI. Du fait de la stimulation de l'oreillette le délai de surveillance ventriculaire est prolongé de la valeur de l'extension du DAV, ce qui correspond toujours au mode AAI.

Lorsque à la fin du DSV prolongé, le stimulateur constate l'absence d'une détection ventriculaire, il stimule le ventricule et bascule automatiquement en mode DDD, ce qui est explicité par la partie droite de la Fig. 2.

Ainsi, en l'absence de détection ventriculaire avant la fin du délai de surveillance ventriculaire (DSV), le stimulateur bascule en mode de fonctionnement DDD, en utilisant les valeurs précédemment calculées pour le DAV de base et le DAV minimal, l'extension du DAV et l'IEA. De cette façon, le basculement en mode DDD s'effectue avec des paramètres optimisés correspondant à l'état du patient au moment du basculement.

Le processus de rebasculement du stimulateur en mode AAI intervient dès que l'une des trois conditions suivantes est satisfaite :
- récupération d'une activité ventriculaire spontanée (détection ventriculaire avant la fin du DAV),
- passage d'une activité auriculaire stimulée à une détection auriculaire (ce qui peut indiquer la fin d'un syndrome vagal),
- après 100 cycles avec stimulation ventriculaire (car le mode DDD automatique est indiqué uniquement pour des troubles paroxystiques de conduction).

Dès que l'une de ces conditions est satisfaite, la valeur calculée pour le DAV est systématiquement augmentée d'une durée comprise entre 16 et 78ms, et de préférence égale à 31ms pour favoriser la récupération de la détection ventriculaire.

Si pendant le DAV prolongé, le stimulateur n'enregistre pas de détection ventriculaire, il stimule le ventricule à la fin de ce délai.

Dans le processus de rebasculement en mode AAI, on peut prévoir une alternative à la troisième condition proposée correspondant à 100 cycles avec stimulation ventriculaire. La troisième condition est alors :
- Si la fréquence de stimulation, ou la fréquence spontanée, devient inférieure ou égale à une fréquence de référence prédéterminée par rapport à la fréquence de base.

Cette fréquence de référence prédéterminée correspond à un intervalle d'échappement ventriculaire égal à l'intervalle d'échappement de base diminué d'une durée égale de préférence à 31ms. Dès que cette condition est satisfaite, la valeur calculée pour le DAV est augmentée de 31ms. Si l'activité ventriculaire spontanée ne réapparait pas, cette extension de 31ms est supprimée dès que la fréquence sinusale redevient supérieure à la fréquence de référence prédéterminée.

Lorsque, pendant 16 cycles consécutifs, le stimulateur détecte des ondes R synchrones des événements auriculaires, il rebascule automatiquement en mode de fonctionnement AAI.

## Revendications

1. Procédé de commande d'un stimulateur cardiaque de type DDD, à commutation automatique de mode de fonctionnement, caractérisé en ce que :
- on contrôle le coeur en mode AAI pendant les périodes de conduction auriculo-ventriculaire ,
- après chaque événement auriculaire hors des périodes réfractaires, on déclenche un délai de surveillance ventriculaire ,
- en cas de défaut de conduction auriculo-ventriculaire pendant ledit délai, on bascule automatiquement en mode DDD et on déclenche une stimulation ventriculaire ,
- après rétablissement de la conduction auriculo-ventriculaire, on rebascule automatiquement en mode AAI.

2. Procédé selon la revendication 1 caractérisé en ce que le délai de surveillance ventriculaire est égal à la moyenne des intervalles entre un événement auriculaire et la détection ventriculaire suivante au cours des cycles précédents augmentée d'une durée prédéterminée, de façon à favoriser la conduction auriculo-ventriculaire spontanée.

3. Procédé selon la revendication 2 caractérisé en ce que le délai de surveillance ventriculaire est égal à la moyenne des intervalles entre un événement auriculaire et la détection ventriculaire suivante au cours des huit cycles précédents, et la durée prédéterminée est comprise entre 16 et 78ms et de préférence égale à 47ms.

4. Procédé selon la revendication 3 caractérisé en ce que le délai de surveillance ventriculaire est limité à 350ms.

5. Procédé selon la revendication 1 caractérisé en ce que lors du basculement en mode DDD, le délai auriculo-ventriculaire est calculé par interpolation linéaire entre une valeur maximale et une valeur minimale, en fonction du rythme sinusal.

6. Procédé selon la revendication 5 caractérisé en ce que les valeurs maximale et minimale du délai auriculo-ventriculaire sont mémorisées pendant les périodes de conduction auriculo-ventriculaire spontanée.

7. Procédé selon la revendication 6, caractérisé en ce que, lorsque la valeur maximale DAVmax est inférieure à la valeur de l'intervalle ER moyen sur un certain nombre de cycles de préférence égal à 8, alors cette valeur DAVmax est augmentée par pas de 16ms.

8. Procédé selon la revendication 6, caractérisé en ce que lorsque, pendant une durée déterminée, typiquement 24h, la valeur DAVmax n'a pas varié, alors elle est diminuée de 16ms.

9. Procédé selon la revendication 6, caractérisé en ce que lorsque la valeur minimale DAVmin est supérieure à la valeur de l'intervalle ER moyen sur un certain nombre de cycles de préférence égal à 8, alors cette valeur DAVmin est diminuée par pas de 16 ms.

10. Procédé selon la revendication 6, caractérisé en ce que lorsque pendant une durée déterminée, typiquement 24 h, la valeur DAVmin n'a pas varié, alors elle est augmentée de 16 ms.

11. Procédé selon la revendication 1 caractérisé en ce que chaque fois que l'on passe d'une détection auriculaire à une stimulation auriculaire, on calcule une extension du délai auriculo-ventriculaire, égale à la différence entre d'une part l'intervalle entre la stimulation auriculaire et la détection ventriculaire suivante, et d'autre part l'intervalle entre la détection auriculaire et la détection ventriculaire suivante, ce dernier intervalle ayant été mesuré au cours de l'antépénultième cycle.

12. Procédé selon la revendication 1, caractérisé en ce que, chaque fois que l'on passe d'une stimulation auriculaire à une détection auriculaire, on calcule une extension du délai auriculo-ventriculaire, égale à la différence entre d'une part l'intervalle entre la stimulation auriculaire et la détection ventriculaire suivante, et d'autre part la détection auriculaire et la détection ventriculaire suivante, le premier intervalle ayant été mesuré au cours de l'antépénultième cycle.

13. Procédé selon la revendication 1 caractérisé en ce que les conditions de rétablissement de la conduction auriculo-ventriculaire sont réunies lorsque l'une des conditions suivantes est réalisée :
- passage d'une activité auriculaire stimulée à une détection auriculaire,
- récupération d'une activité ventriculaire spontanée,
- après cent cycles avec stimulation ventriculaire.

14. Procédé selon la revendication 13 caractérisé en ce que le rebasculement en mode AAI est automatiquement assuré si, pendant seize cycles consécutifs, le stimulateur détecte des ondes R synchrones des ondes P.

15. Procédé selon la revendication 1 caractérisé en ce que lorsque la fréquence de stimulation, ou la fréquence spontanée, devient inférieure ou égale à une fréquence de référence prédéterminée par rapport à la fréquence de base, ladite fréquence de référence prédéterminée correspondant à un intervalle d'échappement égal à l'intervalle d'échappement de base diminué d'une durée égale de préférence à 31 ms, alors pour favoriser le rétablissement de la conduction auriculo-ventriculaire, le délai auriculo-ventriculaire est prolongé d'une durée comprise entre 16 et 78 ms, et de préférence égale à 47 ms.

16. Procédé selon la revendication 1 caractérisé en ce que lors du passage de détection auriculaire à stimulation auriculaire, l'intervalle d'échappement auriculaire est raccourci, pendant huit cycles au plus, d'une valeur programmée, pour accélérer la fréquence de stimulation auriculaire de façon à compenser la chute du débit sanguin.

## Claims

1. A method for controlling a cardiac pulse generator of the DDD type, with automatic operation mode switching, characterized in that :
- one controls the heart in the AAI mode during atrio-ventricular conduction periods,
- after each atrial event outside of the refractory periods, one triggers a ventricular surveillance delay,
- in case of a lack of atrio-ventricular conduction during the said delay, one switches over automatically to the DDD mode and triggers a ventricular pacing,
- after the atrio-ventricular conduction is restored, one reverts automatically to the AAI mode.

2. A method according to claim 1, characterized in that :
- the ventricular surveillance delay equals the average value of intervals between one atrial event and the subsequent ventricular detection during the preceding cycles, increased by a predetermined duration, so as to encourage the spontaneous atrio-ventricular conduction.

3. A method according to claim 2, characterized in that the ventricular surveillance delay equals the average value of intervals between one atrial event and the subsequent ventricular detection during the eight preceding cycles, and the predetermined duration is comprised between 16 and 78 ms and is preferably 47 ms.

4. A method according to claim 3, characterized in that the ventricular surveillance delay is restricted to 350 ms.

5. A method according to claim 1, characterized in that, at the time of switching to the DDD mode, the atrio-ventricular delay is calculated through linear interpolation between a maximum value and a minimum value, as a function of the sinus rhythm.

6. A method according to claim 5, characterized in that the maximum and minimum values of the atrio-ventricular delay are memorized during the periods of spontaneous atrio-ventricular conduction.

7. A method according to claim 6, characterized in that, when the maximum value DAVmax is lower than the value of the average ER interval during a number of cycles preferably equal to 8, then this value DAVmax is increased by steps of 16 ms.

8. A method according to claim 6, characterized in that, when, during a determined duration, typically 24 h, the value DAVmax has not varied, then it is decreased by 16 ms.

9. A method according to claim 6, characterized in that, when the minimum value DAVmin, is higher than the value of the average ER interval during a number of cycles preferably equal to 8, then this value DAVmin is decreased by steps of 16 ms.

10. A method according to claim 6, characterized in that, when, during a determined duration, typically 24 h, the value DAVmin has not varied, then it is increased by 16 ms.

11. A method according to claim 1, characterized in that, upon each change from an atrial detection to an atrial pacing, a calculation is made of an extension of the atrio-ventricular delay being equal to the difference between on the one hand the interval between the atrial pacing and the next ventricular detection, and on the other hand the interval between the atrial detection and the next ventricular detection, this latter interval being measured during the antepenultimate cycle.

12. A method according to claim 1, characterized in that, upon each change from an atrial pacing to an atrial detection, a calculation is made of an extension of the atrio-ventricular delay being equal to the difference between, on the one hand, the interval between the atrial pacing and the next ventricular detection, and on the other hand, the interval between the atrial detection and the next ventricular detection, said first interval being measured during the antepenultimate cycle.

13. A method according to claim 1, characterized in that the restoration of the atrio-ventricular conduction is considered as achieved when one of the following conditions is met :
- change from a paced atrial activity to an atrial detection,
- recovery of a spontaneous ventricular activity,
- after one hundred cycles with ventricular pacing.

14. A method according to claim 13, characterized in that the reversal to the AAI mode is automatically ensured if, during sixteen consecutive cycles, the pulse generator detects R waves which are synchronous with the P waves.

15. A method according to claim 1 characterized in that when the pacing rate, or spontaneous rate, becomes lower than or equal to a reference rate predetermined with respect to basic rate, said predetermined reference rate corresponding with an escape interval equal to the basic escape interval shortened by a duration preferably equal to 31 ms, then to encourage the restoration of the atrio-ventricular conduction, the atrio-ventricular delay is extended by a duration between 16 and 78 ms, preferably equal to 47 ms.

16. A method according to claim 1, characterized in that, upon changing from atrial detection to atrial pacing, the atrial escape interval is shortened, during eight cycles, at most, by a programmed value, for accelerating the atrial pacing rate, so as to compensate the drop of the cardiac output.

## Patentansprüche

1. Verfahren zur Steuerung eines mit automatischer Umschaltung der Betriebsart versehenen Herzschrittmachers vom Typ DDD,
**dadurch gekennzeichnet, daß**
- das Herz während der Perioden der aurikulär-ventrikulären Leitung in der AAI-Betriebsart gesteuert wird,
- nach jedem aurikulären Ereignis außerhalb der Refraktärphasen eine ventrikuläre Überwachungszeit ausgelöst wird,
- im Fall des Versagens der aurikulär-ventrikulären Leitung während dieser Zeit automatisch in die DDD-Betriebsart gewechselt wird und eine ventrikuläre Stimulation ausgelöst wird,
- nach dem Wiederherstellen der aurikulär-ventrikulären Leitung automatisch in die AAI-Betriebsart zurückgewechselt wird.

2. Verfahren nach Anspruch 1,
**dadurch gekennzeichnet, daß**
die ventrikuläre Überwachungszeit gleich dem Mittelwert der Intervalle zwischen einem aurikulären Ereignis und der folgenden ventrikulären Erfassung während der vorhergehenden Zyklen ist, der um eine vorbestimmte Dauer erhöht wird, so daß die spontane aurikulär-ventrikuläre Leitung begünstigt wird.

3. Verfahren nach Anspruch 2,
**dadurch gekennzeichnet, daß**
die ventrikuläre Überwachungszeit gleich dem Mittelwert der Intervalle zwischen einem aurikulären Ereignis und der folgenden ventrikulären Erfassung während der vorhergehenden acht Zyklen ist, und die vorbestimmte Dauer zwischen 16 und 78 ms liegt und vorzugsweise gleich 47 ms ist.

4. Verfahren nach Anspruch 3,
**dadurch gekennzeichnet, daß**
die ventrikuläre Überwachungszeit auf 350 ms begrenzt ist.

5. Verfahren nach Anspruch 1,
**dadurch gekennzeichnet, daß**
bei dem Umschalten in die DDD-Betriebsart die aurikulär-ventrikuläre Verzögerung in Abhängigkeit des sinusbezüglichen Rhythmus mittels einer linearen Interpolation zwischen einem Maximalwert und einem Minimalwert berechnet wird.

6. Verfahren nach Anspruch 5,
**dadurch gekennzeichnet, daß**
die Maximalwerte und Minimalwerte der aurikulär-ventrikulären Verzögerung während der Perioden der spontanen aurikulär-ventrikulär Leitung gespeichert werden.

7. Verfahren nach Anspruch 6,
**dadurch gekennzeichnet, daß**
dann, wenn der Maximalwert DAVmax kleiner ist als der Wert des Intervalls ER, gemittelt über eine gewisse Anzahl von Zyklen, vorzugsweise acht, dieser Wert DAVmax schrittweise um 16 ms erhöht wird.

8. Verfahren nach Anspruch 6,
**dadurch gekennzeichnet, daß,**
wenn sich der Wert DAVmax während einer bestimmten Dauer, typischerweise 24 h, nicht verändert hat, dieser um 16 ms verringert wird.

9. Verfahren nach Anspruch 6,
**dadurch gekennzeichnet, daß**
dann, wenn der Minimalwert DAVmin größer ist als der Wert des Intervalls ER, gemittelt über eine gewisse Anzahl von Zyklen, vorzugsweise acht, dieser Wert DAVmin schrittweise um 16 ms verringert wird.

10. Verfahren nach Anspruch 6,
**dadurch gekennzeichnet, daß,**
wenn sich der Wert DAVmin während einer bestimmten Dauer, typischerweise 24 h, nicht verändert hat, dieser um 16 ms erhöht wird.

11. Verfahren nach Anspruch 1
**dadurch gekennzeichnet, daß**
jedesmal, wenn von einer aurikulären Erfassung auf eine aurikuläre Stimulation übergegangen wird, eine Ausdehnung der aurikulär-ventrikulären Verzögerung berechnet wird, die gleich der Differenz zwischen einerseits dem Intervall zwischen der aurikulären Stimulation und der darauffolgenden ventrikulären Erfassung, und andererseits dem Intervall zwischen der aurikulären Erfassung und der darauffolgenden ventrikulären Erfassung ist, wobei das letzte Intervall während dem drittletzten Zyklus gemessen wurde.

12. Verfahren nach Anspruch 1
**dadurch gekennzeichnet, daß**
jedesmal, wenn von einer aurikulären Stimulation auf eine aurikuläre Erfassung übergegangen wird, eine Ausdehnung der aurikulär-ventrikulären Verzögerung berechnet wird, die gleich der Differenz zwischen einerseits dem Intervall zwischen der aurikulären Stimulation und der darauffolgenden ventrikulären Erfassung, und andererseits der aurikulären Erfassung und der darauffolgenden ventrikulären Erfassung ist, wobei das erste Intervall während dem drittletzten Zyklus gemessen wurde.

13. Verfahren nach Anspruch 1
**dadurch gekennzeichnet, daß**
die Bedingungen zur Wiederherstellung der aurikulär-ventrikulären Leitung immer dann erfüllt sind, wenn eine der folgenden Bedingungen erfüllt ist:
- Übergang von einer stimulierten aurikulären Aktivität zu einer aurikulären Erfassung,
- Erholung von einer spontanen ventrikulären Aktivität
- nach hundert Zyklen mit ventrikulärer Stimulation.

14. Verfahren nach Anspruch 13,
**dadurch gekennzeichnet, daß**
das Zurückschalten in die AAI-Betriebsart automatisch sichergestellt ist, wenn der Schrittmacher während sechzehn aufeinanderfolgenden Zyklen die R-Zacken synchron zu den P-Zacken erfaßt.

15. Verfahren nach Anspruch 1
**dadurch gekennzeichnet, daß**
dann, wenn die Stimulations-Frequenz oder die spontane Frequenz kleiner oder gleich einer mit Bezug auf die Grundfrequenz vorbestimmten Bezugsfrequenz wird, wobei die vorbestimmte Bezugsfrequenz, die einem Ausströmintervall entspricht, das gleich dem um eine Dauer von vorzugsweise gleich 31 ms verringerten Basis-Ausströmintervall ist, um die Wiederherstellung der aurikulär-ventrikulären Leitung zu begünstigen, die aurikulär-ventrikuläre Verzögerung um eine Dauer zwischen 16 und 78 ms, vorzugsweise 47 ms, verlängert wird.

16. Verfahren nach Anspruch 1
**dadurch gekennzeichnet, daß**
bei dem Übergang von der aurikulären Erfassung zur aurikulären Stimulierung das aurikuläre Ausströmintervall während acht Zyklen mehr um einen programmierten Wert verkürzt wird, um die aurikuläre Stimulierungsfrequenz zu beschleunigen, so daß das Absinken des Herzblutausstoßes kompensiert wird.
